# EUROPEAN PATENT SPECIFICATION

(11) **EP 0 904 099 B1**
(45) Date of publication and mention of the grant of the patent: **16.11.2005**
(21) Application number: 97918073.4
(22) Date of filing: 24.04.1997
(51) Int. Cl.: A61K 38/27, A61K 47/18, A61P 43/00

(54) **PHARMACEUTICAL FORMULATION CONTAINING HUMAN GROWTH HORMONE, HISTIDINE AND A NON-IONIC DETERGENT**
PHARMAZEUTISCHE FORMULIERUNG, BESTEHEND AUS MENSCHLICHEM WACHSTUMSHORMON, HISTIDINE UND EINEM NICHTIONISCHEN DETERGENZ
FORMULATION PHARMACEUTIQUE CONTENANT L'HORMONE DE CROISSANCE HUMAINE, L'HISTIDINE ET UN DETERGENT NON IONIQUE

(30) Priority: 24.04.1996 DK 49096
(43) Date of publication of application: 31.03.1999
(73) Proprietor: NOVO NORDISK A/S, 2880 Bagsvaerd (DK)
(72) Inventor: BJOERN, Soeren, DK-2800 Lyngby (DK); SOERENSEN, Hans, Holmegaard, DK-2830 Virum (DK); LANGBALLE, Peter, DK-2920 Charlottenlund (DK); LARSEN, Silke, Moeller, DK-2920 Charlottenlund (DK); EBBEHOEJ, Kirsten, DK-2850 Naerum (DK); HANSEN, Birthe, Lykkegaard, DK-3500 Vaerloese (DK)
(86) International application number: PCT/DK1997/000184
(87) International publication number: WO 1997/039768

(56) References cited:
- WO-A-89/09614
- WO-A-93/12812

## Description

### FIELD OF THE INVENTION

The invention relates to novel aqueous pharmaceutical formulations comprising human growth hormone, histidine, and a non-ionic detergent, viz. poloxamer 188. The invention further relates to a method of producing such formulations, and to the use of such formulations in the manufacture of medicaments for treating disorders affectable by human growth hormone.

### BACKGROUND OF THE INVENTION

The growth hormones from man and from the common domestic animals are proteins of approximately 191 amino acids, synthesized and secreted from the anterior lope of the pituitary gland. Human growth hormone consists of 191 amino acids.

Growth hormone is a key hormone involved in the regulation of not only somatic growth, but also in the regulation of metabolism of proteins, carbohydrates and lipids. The major effect of growth hormone is to promote growth.

The organ systems affected by growth hormone include the skeleton, connective tissue, muscles, and viscera such as liver, intestine, and kidneys.

Until the development of recombinant technology and the cloning of the growth hormone gene now giving rise to production of e.g. human growth hormone (hGH) and Met-hGH in industrial scale, human growth hormone could only be obtained by extraction from the pituitary glands of human cadavers. The very limited supplies of growth hormone restricted the use thereof to longitudinal growth promotion in childhood and puberty for treatment of dwarfism, even though it has been proposed for, inter alia, treatment of short stature (due to growth hormone deficiency, normal short stature and Turner syndrome), growth hormone deficiency in adults, infertility, treatment of bums, wound healing, dystrophy, bone knitting, osteoporosis, diffuse gastric bleeding, and pseudoarthrosis.

Furthermore, growth hormone has been proposed for increasing the rate of growth of domestic animals or for decreasing the proportion of fat.

Pharmaceutical preparations of growth hormone tend to be unstable. Degradation products such as deamidated or sutfoxydated products and dimer or polymer forms are generated - especially in solutions of growth hormone.

The predominant chemical degradation reactions of hGH are 1) deamidation by direct hydrolysis or via a cyclic succinimide intermediate to form various amounts of L-asp-hGH, L-iso-asp-hGH, D-asp-hGH, and D-iso-asp-hGH (ref 1-3), 2) oxidation of the methionine residues in positions 14 and 125 (ref 4-9), and 3) cleavage of peptide bonds.

Deamidation especially takes place at the Asn in position 149.

hGH is rather easily oxidized in positions 14 and 125, especially in solution (4-8), since the oxidation of hGH in solution forming sulfoxides is normally due to the oxygen dissolved in the preparation.

At present, it is not believed that these degradation products should have toxic or altered biological activity or receptor binding properties, but there is indication to the effect that the conformation stability of the sulfoxides is reduced as compared to native hGH.

Other "degradation products" of growth hormone are aggregation products such as dimers and polymers. Studies have been performed to clarify the role of these forms in inducing an immune response with measurable amounts of antibodies to native hGH. These studies have indicated that aggregates of hGH are the primary cause of immunogenicity in patients.

Thus, it is desirable in a pharmaceutical formulation to avoid the formation of aggregates of growth hormone since such aggregates are capable of causing undesirable immunogenicity or altered half-life.

The kinetics of degradation depend on temperature, pH and various additives or adjuvants in the hGH formulation.

Due to the instability, a growth hormone formulation is at present normally lyophilized and stored in the lyophilized form at 2-8°C until it is reconstituted for use in order to minimize the degradation. The lyophilized pharmaceutical preparations comprising hGH are reconstituted by the patient and then stored as a solution during the use for a period of up to 30 days, during which some degradation will take place.

It is at present preferred to reconstitute the growth hormone as late as possible before use and to store and ship the preparation in a lyophilized state. The chain from the manufacturer to the pharmacy is apt for handling the preparations at a controlled low temperature of e.g. 2-8°C which allows for a shelf life of up to three years.

Preferably, a lyophilized and then reconstituted preparation should be stable with the end user in a lyophilized state for about one month and additionally for one month in a reconstituted state in a pen device for the intended period of use of a cartridge.

As an alternative to lyophilized and reconstituted preparations, growth hormone may be formulated as a liquid formulation suitable for use in vials or in a pen system for self-medication. The extended use of pen systems for self-medication and the expanded field of use calls for a preparation which is stable for a sufficient long time with the end user. Such stabilization is of very great importance when moving the administration of the growth hormone from clinics to the homes of the individuals to be treated, where optimal storage conditions may not be available. A "ready to use" formulation furthermore diminishes any handling problems in connection with reconstitution and thus represents a convenience for the patient.

A stable dissolved preparation comprising growth hormone may be produced ready to use in the form of vials used by the patient in combination with conventional syringes, or as cartridges fitting into the pen device used by the patient. In both cases, the patient may then avoid the reconstitution of the preparation and, hence, will not have to be in the possession of a lyophilized preparation, a suitable vehicle for reconstitution as well as the necessary skill and sterile equipment for sterile reconstitution of the preparation. Safety reasons also make it desirable to avoid the reconstitution of a lyophilized preparation just before the use of the preparation.

From the manufacturer's point of view, it may be an advantage to avoid the lyophilization step in the production of growth hormone preparations. Lyophilization is a time consuming and costly process and is also often a "bottleneck" in the production due to the limited capacity of the freeze drier.

Thus, there is a need for more stable dissolved preparations of growth hormone in order to facilitate the handling to be performed by the patient.
Thus, there is also a need to reduce the rate of the degradation processes in order to allow for dissolved hGH preparations being stable during shelf life and during the period of use of up to about one month.

### Prior art

Prior attempts to stabilize hGH have not fully succeeded in preventing the formation of dimer. The problems associated with dimer formation are, e.g, noted in Becker, G.W., Biotechnology and Applied Biochemistry **9**, 478 (1987).

International Patent Publication No. WO 89/09614 and Australian patent application No. 30771/89 disclose a stable pharmaceutical formulation containing human growth hormone, glycine, and mannitol. Such a formulation shows improved stability during normal processing and storage in a lyophilized state as well as in the period of use after the reconstitution.

Published European patent application No. 303 746 discloses that animal growth hormone may be stabilized with various stabilizers to give decreased formation of insolubles and preservation of the soluble activity in aqueous environments, such stabilizers including certain polyols, amino acids, polymers of amino acids having a charged side group at physiological pH, and choline salts. Polyols are selected from the group consisting of non-reducing sugars, sugar alcohols, sugar acids, pentaerythritol, lactose, water-soluble dextrans and Ficoll: amino acids are selected from the group consisting of glycine, sarcosine, lysine or salts thereof, serine, arginine or salts thereof, betaine, N,N,-dimethyl-glycine, aspartic acid or salts thereof, glutamic acid or salts thereof; a polymer of an amino acid having a charged side group at physiological pH may be selected from polylysine, polyaspartic acid, polyglutamic acid, polyarginine, polyhistidine, polyomithine and salts thereof; and choline derivatives are selected from the group consisting of choline chloride, choline dihydrogen citrate, choline bitartrate, choline bicarbonate, tricholine citrate, choline ascorbate, choline borate, choline gluconate, choline phosphate, di(choline)sulphate and dicholine mucate.

US patent specification No. 4,917,685 discloses a delivery device designed to be implanted comprising growth hormone stabilized using the same stabilizers as mentioned in EP 303746.

Published European patent application No. 374,120 discloses a stabilized formulation comprising hGH and a polyol having three hydroxy groups. Glycerol and tris(hydroxymethyl)aminomethane are mentioned. Furthermore, the presence of histidine hydrochloride as a buffer together with the polyol is disclosed.

International Patent Publication No. WO 93/12811 discloses stabilized formulations of growth hormone in the form of a lyophilized powder or an aqueous solution - comprising asparagine.

International Patent Publication No. WO 93/12812 discloses stabilized formulations of growth hormone in the form of a lyophilized powder or an aqueous solution comprising histidine. In such formulations the deamidation is reduced by 25-30% as compared to a corresponding formulation of growth hormone comprising phosphate buffer.

International patent Publication No. WO 96/11703 discloses stabilized formulations of growth hormone comprising isoleucine.

International patent Publication No. WO 96/11702 discloses stabilized formulations of growth hormone comprising valine.
International patent Publication No. WO 96/11704 discloses stabilized formulations of growth hormone comprising leucine.

International Patent Publication No. WO 93/19776 discloses protein formulations comprising growth hormone comprising citrate as buffer substance being more stable than formulations comprising phosphate buffer. The formulations may also comprise amino acids such as glycine and alanine and/or mannitol or other sugar alcohols and/or glycerol and/or other carbohydrates and optionally a preservative such as benzyl alcohol.

International Patent Publication No. WO 94/03198 discloses a stable aqueous formulation containing human growth hormone, a buffer, a non-ionic surfactant, and, optionally a neutral salt, mannitol, or, a preservative. The buffer may be histidine, although citrate is preferred. The application discloses a preferred formulation containing citrate as buffer, and natrium chloride and polysorbate 20 (Tween 20) for stabilization. This combination stabilizes the said aqueous formulation against aggregation but does give rise to a considerate amount of deamidated growth hormone.

International patent Publication No. WO 95/35116 discloses a stable lyophilized formulation containing saccharose and, optionally, mannitol.

### DESCRIPTION OF THE INVENTION

It has now surprisingly been found that an aqueous pharmaceutical formulation comprising human growth hormone (hGH) in an amount of from 1 mg/ml to 15 mg/ml, histidine in an amount of from 0.05 to 0.5 mg per mg of hGH, poloxamer 188 in an amount of from 0.1 to 2 mg per mg hGH, mannitol in an isotonicity-conferring amount and phenol as a preservative, and having a pH of from 6.0 to 6.3, shows high stability against deamidation and aggregation. Preferred embodiments of aqueous formulations of the invention have a pH of from 6.0 to 6.2, suitably about 6.1.

A further aspect of the invention relates to a method for preparing an aqueous pharmaceutical formulation according to the invention, the method comprising dissolving hGH, in an amount that results in an amount thereof in the final aqueous formulation of from 1 mg/ml to 15 mg/ml, in an aqueous solution comprising:
deionized water;
histidine in an amount that results in an amount thereof in the final aqueous formulation of from 0.05 to 0.5 mg per mg of hGH;
poloxamer 188 in an amount that results in an amount thereof in the final aqueous formulation of from 0.1 to 2 mg per mg hGH;
mannitol in an amount that results in an isotonicity-conferring amount thereof in the final aqueous formulation; and
phenol;
wherein the pH of the resulting formulation is adjusted to have a value of from 6.0 to 6.3.

In preferred embodiments of the method of the invention, the pH of the resulting formulation is adjusted to have a value of from 6.0 to 6.2, suitably a pH of about 6.1.

The pH of the formulation may be adjusted by adding an acid which has no adverse effect on the growth hormone, preferably a physiologically acceptable acid, e.g. a mineral acid such as hydrochloric acid, sulphuric acid or nitric acid, or an organic acid such as acetic acid or citric acid.

A still further aspect of the invention relates to the use of an aqueous pharmaceutical formulation according to the invention in the manufacture of a medicament for treating a disorder affectable by human growth hormone.

Aqueous pharmaceutical formulations of the invention may be formulated for administration in any suitable manner, e.g. by parenteral administration or administration to a mucosal membrane, such as by nasal administration. The formulation may be presented in the form of a dose contained in a vial or cartridge or any other suitable container, such as a prefilled syringe or a pen device.

The human growth hormone used in accordance with the invention may be native growth hormone isolated, e.g. by extracting pituitary glands in a conventional manner, or growth hormone produced by recombinant techniques, e.g as described in E. B. Jensen and S. Carlsen in Biotech and Bioeng. 36. 1-11 (1990). The growth hormone may be a concentrate obtained directly from the fermentation broth or a conventional lyophilized preparation which is dissolved in an appropriate solvent.

The term "dose" of growth hormone refers to that amount that provides therapeutic effect in an administration regimen. The formulations hereof are prepared containing amounts of hGH of from about 1 mg/ml to about 15 mg/ml, e.g. from 1 mg/ml to about 10 mglml, calculated on the ready-to-use formulation. For use of these compositions in administration to human beings suffering from hypopituitary dwarfism, for example, these formulations contain from about 0.1 mg/ml to about 10 mg/ml, corresponding to the currently contemplated dosage regimen for the intended treatment. The concentration range is not critical to the invention and may be varied by the physician supervising the administration.

The invention is explained further in the Examples below, which illustrate the invention.

### EXPERIMENTAL PART

### Example 1:

### Inhibition of aggregation

Liquid formulations of hGH were prepared by dissolving hGH in a solution of excipients and adjusting the pH with HCl/NaOH. The composition of the formulations were:

| | |
|---|---|
| hGH | 5 mg/ml |
| Mannitol | 45 mg/ml |
| L-histidin | 0.62 mg/ml |
| Phenol | 2,5 mg/ml |
| Surfactant | x mg/ml |

pH = y (from 6.1 to 6.8)
The appearance of the solutions was examined visually after the solutions had been stored 1 day at 8°C and rotated for 19 hour (20 rpm) at 25°C, respectively:

| **Surfactant** | **x (mg/ml)** | **pH (y)** | **Apperance after 1 day at 8°C** | **Appearance after 19 hours rotation** |
|---|---|---|---|---|
| - | 0 | 6.1 | Precipitation | Precipitation |
| - | 0 | 6.3 | Precipitation | Many flakes |
| - | 0 | 6.5 | Flakes | Many flakes |
| - | 0 | 6.8 | Flakes | Some flakes |
| Pluronic L64 | 0.5 | 6.8 | Clear, few flakes | Many small flakes |
| Pluronic L64 | 1 | 6.8 | Clear, few flakes | Clear, some flakes |
| Pluronic F68 | 2 | 6.1 | Clear | Clear, some flakes |
| Pluronic F68 | 2 | 6.3 | Clear | Clear, few flakes |
| Pluronic F68 | 2 | 6.5 | Clear | Clear, few flakes |
| Pluronic F68 | 2 | 6.8 | Clear | Clear, some flakes |
| Lutrol F127 | 1 | 6.1 | Clear | Clear, some flakes |
| Lutrol F127 | 1 | 6.3 | Clear | Clear, some flakes |
| Lutrol F127 | 1 | 6.5 | Clear | Clear, few flakes |
| Lutrol F127 | 1 | 6.8 | Clear | Not analysed |
| Tween 20 | 2 | 6.1 | Slight precipitation | Reasonably clear, some flakes |
| Tween 20 | 2 | 6.3 | Clear | Clear, some flakes |
| Tween 20 | 2 | 6.5 | Clear, some flakes | Clear, few flakes |
| Tween 20 | 2 | 6.8 | Clear | Clear, some flakes |

### Example 2:

### Rate of deamidation

The eight formulations tabulated below were prepared by adding the hGH to a solution containing the excipients. pH was adjusted to 6.1. The preparations were then placed for 28 days at 25°C and for 14 days at 37°C and analysed for formation of deamidated forms of hGH. Rate constants for the deamidation were calculated on basis of the stability data. In the table below the contents of hGH and excipients are given in mg/ml and the rate constants k_{25°C} and k_{37°C} are given in days⁻¹

| hGH | Phenol | Pluronic F68 | Lutrol F127 | L-histidin | Di-sodium hydrogen citrate | Mannitol | NaCl | k_{25°C} | k_{37°C} |
|---|---|---|---|---|---|---|---|---|---|
| 5 | 2.5 | 2 | | 0.62 | | 45 | | 0.00180 | 0.00860 |
| 6.7 | 2.5 | 2 | | | 2.2 | 45 | | 0.00221 | 0.0127 |
| 6.7 | 2.5 | 2 | | 0.62 | | | 5.8 | 0.00244 | 0.0110 |
| 6.7 | 2.5 | 2 | | | 2.2 | | 5.8 | 0.00263 | 0.0143 |
| 5 | 2.5 | | 1 | 0.62 | | 45 | | 0.00178 | 0.00914 |
| 6.7 | 2.5 | | 1 | | 2.2 | 45 | | 0.00221 | 0.0128 |
| 6.7 | 2.5 | | 1 | 0.62 | | | 5.8 | 0.00236 | 0.0112 |
| 6.7 | 2.5 | | 1 | | 2.2 | | 5.8 | 0.00259 | 0.0139 |

### Example 3:

### Formation of deamidated forms

The formulations tabulated below were prepared by adding the hGH to a solution containing the excipients and adjusting the pH. The preparations were then placed for 3 months at 8°C and 25°C and analysed for formation of deamidated forms of hGH.

| **hGH (mg/ml)** | **Surfactant** ^{**1)**} **(mg/ml)** | **Phenol (mg/ml)** | **L-histidin (mg/ml)** | **Mannitol (mg/ml)** | **pH** | **Content of desamido forms (%)** | | | |
|---|---|---|---|---|---|---|---|---|---|
| | | | | | | **t=0** | **3 months at 8°C** | **12 weeks at 25°C** | **3 weeks at 37°C** |
| 6.25 | 2 (F68) | 2.5 | 0.62 | 45 | 6.3 | 0.6 | 2.7 | 18.1 | 21.1 |
| 6.25 | 2 (F68) | 2.5 | 0.62 | 45 | 6.2 | 0.5 | 2.5 | 16.2 | 18.7 |
| 6.25 | 2 (F68) | 2.5 | 0.62 | 45 | 6.1 | 0.6 | 2.2 | 14.8 | 17.1 |
| 3.33 | 3 (F68) | 3.0 | 0.68 | 40 | 6.1 | 1.3 | 3.0 | 15.5 | n.a. |
| 6.67 | 3 (F68) | 3.0 | 0.68 | 40 | 6.1 | 1.0 | 3.2 | 16.6 | n.a. |
| 10.0 | 3 (F68) | 3.0 | 1.1 | 39 | 6.1 | 0.6 | 3.8 | 16.7 | n.a. |
| 8.25 | 1 (L127) | 2.5 | 0.62 | 45 | 6.1 | 0.6 | 2.5 | 15.4 | 18.2 |
| 6.25 | 2 (T20) | 2.5 | 0.62 | 45 | 6.1 | 0.6 | n.a. | n.a. | 16.6 |
| 1): Type of surfactant is given in parentheses: | | | | | | | | | |
| F68 = Pluronic F68 = poloxamer 188 | | | | | | | | | |
| F127 = Lutrol 127 = poloxamer 407 | | | | | | | | | |
| T20 = Tween 20 = polysorbate 20 | | | | | | | | | |

### Example 4:

24 formulations were prepared as follows:

| | |
|---|---|
| hGH | 3.33 to 13.1 mg/ml |
| L-histidin | 0.78 mg/ml |
| Mannitol | 22 mg/ml |
| Sucrose | 21 mg/ml |
| Poloxamer 188 | 1.33 - 5.33 mg/ml |
| Preservative: | Benzylalcohol (0 - 20 mg/ml) or Phenol (0-5 mg/ml) |

The formulations were prepared by adding the hGH to a solution of the excipients. pH was adjusted to 6.8. The formulations were kept at 25°C for 4 weeks and then the clarity of the solutions were measured as the absorbance at 340 nm. The absorbance at 340 nm indicates the degree of aggregation in the solutions.

| | | Benzylalcohol (mg/ml) | | | | Phenol (mg/ml) | |
|---|---|---|---|---|---|---|---|
| hGH (mg/ml) | Poloxamer 188 (mg/ml) | 0 | 15 | 18 | 20 | 2.5 | 5 |
| 3,33 | 1,33 | 0,009 | 0,019 | 0,045 | 0,016 | 0,015 | 0,020 |
| 6,67 | 2,66 | 0,046 | 0,022 | 0,041 | 1) | 0,025 | 0,026 |
| 10,0 | 4,0 | 0,022 | 0,060 | 1) | 1) | 0,034 | 0,050 |
| 13,1 | 5,33 | 0,018 | 0,041 | 0,042 | 1) | 0,054 | 0,071 |
| 1): dissolution of hGH not possible. | | | | | | | |

### REFERENCES

1) Y.-C.J. Wang and M.A. Hanson. Parenteral Formulations of Proteins and Peptides: Stability and Stabilizers. J. Parenteral Science and Technology 42 (Suppl.) (1988) 53-525.
2) M.C. Manning, K. Patel, R.T. Borchardt. Stability of Protein Pharmaceuticals. Pharmaceutical Research 6 (11) (1989) 903-918.
3) B.A. Johnson, J.M. Shirokawa, W.S. Hancock, M.W. Spellman, L.J. Basa and D.W. Asward. J.Biol.Chem. 264, 1462-71 (1989).
4) L.C. Teh et al., J.Biol.Chem., 262, 785-794 (1987).
5) G.W. Becker et al., Biotech.Appl.Biochem., 10, 326-337 (1988).
6) R.A. Houghten et al., Arch.Biochem.Biophys., 178, 350-355 (1977).
7) R.M. Riggin et al., Anal.Biochem., 167, 199-209 (1987).
8) P. Gellerfors et al., Acta Paediatr.Scand (suppl), 370, 93-100 (1990).
9) M.J. Kaufman, Pharm.Res., 7 (3) 289-292 (1990).

## Claims

1. An aqueous pharmaceutical formulation comprising:
human growth hormone (hGH) in an amount of from 1 mg/ml to 15 mg/ml;
histidine in an amount of from 0.05 to 0.5 mg per mg of hGH;
poloxamer 188 in an amount of from 0.1 to 2 mg per mg hGH;
mannitol in an isotonicity-conferring amount;
and
phenol as a preservative;
said formulation having a pH of from 6.0 to 6.3.

2. A formulation according to claim 1 having a pH of about 6.1.

3. A method for preparing an aqueous pharmaceutical formulation according to claim 1, comprising dissolving hGH, in an amount that results in an amount thereof in the final aqueous formulation of from 1 mg/ml to 15 mg/ml, in an aqueous solution comprising:
deionized water;
histidine in an amount that results in an amount thereof in the final aqueous formulation of from 0.05 to 0.5 mg per mg of hGH;
poloxamer 188 in an amount that results in an amount thereof in the final aqueous formulation of from 0.1 to 2 mg per mg hGH;
mannitol in an amount that results in an isotonicity-conferring amount thereof in the final aqueous formulation; and
phenol;
wherein the pH of the resulting formulation is adjusted to have a value of from 6.0 to 6.3.

4. A method according to claim 3, wherein the pH of the resulting formulation is adjusted to have a value of about 6.1.

5. Use of an aqueous pharmaceutical formulation according to claim 1 or 2 in the manufacture of a medicament for treating a disorder affectable by human growth hormone.

## Patentansprüche

1. Wässrige pharmazeutische Formulierung, umfassend:
menschliches Wachstumshormon (hGH) in einer Menge von 1 mg/ml bis 15 mg/ml;
Histidin in einer Menge von 0,05 bis 0,5 mg pro mg hGH;
Poloxamer 188 in einer Menge von 0,1 bis 2 mg pro mg hGH;
Mannit in einer Isotonie-verleihenden Menge;
und
Phenol als Konservierungsmittel;
wobei die Formulierung einen pH-Wert von 6,0 bis 6,3 aufweist.

2. Formulierung nach Anspruch 1, mit einem pH-Wert von etwa 6,1.

3. Verfahren zur Herstellung einer wässrigen pharmazeutischen Formulierung nach Anspruch 1, umfassend das Lösen von hGH in einer Menge, die in der fertigen wässrigen Formulierung zu einer Menge davon von 1 mg/ml bis 15 mg/ml führt, in einer wässrigen Lösung, umfassend
deionisiertes Wasser;
Histidin in einer Menge, die in der fertigen wässrigen Formulierung zu einer Menge davon von 0,05 mg bis 0,5 mg pro mg hGH führt;
Poloxamer 188 in einer Menge, die in der fertigen wässrigen Formulierung zu einer Menge davon von 0,1 mg bis 2 mg pro mg hGH führt;
Mannit in einer Menge, die in der fertigen wässrigen Formulierung zu einer Isotonie-verleihenden Menge davon führt; und
Phenol;
wobei die resultierende Formulierung auf einen Wert von 6,0 bis 6,3 eingestellt wird.

4. Verfahren nach Anspruch 3, wobei der pH-Wert der resultierenden Formulierung auf einen pH-Wert von etwa 6,1 eingestellt wird.

5. Verwendung einer wässrigen pharmazeutischen Formulierung nach Anspruch 1 oder 2 bei der Herstellung eines Medikaments zur Behandlung einer Störung, die durch menschliches Wachstumshormon beeinflussbar ist.

## Revendications

1. Une formulation pharmaceutique aqueuse comprenant :
de l'hormone de croissance humaine (hGH) à raison de 1 mg/ml à 15 mg/ml ;
de l'histidine à raison de 0,05 à 0,5 mg par mg de hGH ;
du poloxamer 188 à raison de 0,1 à 2 mg par mg de hGH ;
du mannitol selon une quantité procurant de l'isotonicité
et
du phénol en tant que conservateur ;
ladite formulation ayant un pH de 6,0 à 6,3.

2. Une formulation selon la revendication 1 ayant un pH d'environ 6,1.

3. Un procédé de préparation d'une formulation pharmaceutique aqueuse selon la revendication 1, comprenant la dissolution de hGH selon une quantité qui conduit à une quantité de celle-ci dans la formulation aqueuse finale de 1 mg/ml à 15 mg/ml, dans une solution aqueuse comprenant :
de l'eau désionisée ;
de l'histidine selon une quantité qui conduit à une quantité de celle-ci dans la formulation aqueuse finale de 0,05 à 0,5 mg par mg de hGH ;
du poloxamer 188 selon une quantité qui conduit à une quantité de celui-ci dans la formulation aqueuse finale de 0,1 à 2 mg par mg de hGH ;
du mannitol selon une quantité qui conduit à une quantité de celui-ci procurant de l'isotonicité dans la formulation aqueuse finale
et
du phénol ;
dans lequel le pH de la formulation résultante est ajusté pour avoir une valeur de 6,0 à 6,3.

4. Un procédé selon la revendication 3, dans lequel le pH de la formulation résultante est ajusté pour avoir une valeur d'environ 6,1.

5. Utilisation d'une formulation pharmaceutique aqueuse selon la revendication 1 ou 2 dans la fabrication d'un médicament pour traiter un désordre apte à être affecté par l'hormone de croissance humaine.
